# EUROPÄISCHE PATENTANMELDUNG

(11) **EP 4 201 900 A2**
(43) Veröffentlichungstag der Anmeldung: **28.06.2023**
(21) Anmeldenummer: 22212836.5
(22) Anmeldetag: 12.12.2022
(51) Int. Cl.: C03C 10/00, C03C 4/00, C03C 3/083, A61K 6/807, A61K 6/809, A61K 6/822, A61K 6/824, A61K 6/833, C03C 3/085, C03C 3/087, C03C 3/091, C03C 3/095, C03C 3/097, C03C 3/112

(54) **LITHIUMSILIKAT-GLASKERAMIK MIT GEHALT AN ZINN**

(30) Priorität: 23.12.2021 EP 21217422
(71) Anmelder: Ivoclar Vivadent AG, 9494 Schaan (LI)
(72) Erfinder: Dittmer, Marc, 6800 Feldkirch (AT); Ritzberger, Christian, 9472 Grabs (CH); Rampf, Markus, 7212 Seewis-Dorf (CH); Sulser, Katrin, 9477 Trübbach (CH)
(74) Vertreter: Uexküll & Stolberg

(57) **Zusammenfassung**

Es werden Lithiumsilikat-Glaskeramiken und Vorstufen davon beschrieben, die Zinn enthalten und sich durch sehr gute mechanische und optische Eigenschaften auszeichnen und insbesondere als Restaurationsmaterial in der Zahnheilkunde eingesetzt werden können.

## Beschreibung

Die Erfindung betrifft Lithiumsilikat-Glaskeramik, die Zinn enthält, und sich insbesondere zum Einsatz in der Zahnheilkunde und bevorzugt zur Herstellung von dentalen Restaurationen eignet, sowie Vorstufen zur Herstellung dieser Glaskeramik.

Glaskeramiken mit Gehalt an Zinn sind aus dem Stand der Technik bekannt.

Die EP 1 985 591 beschreibt Glaskeramiken, die durch Metallkolloide eingefärbt sein können. Als Metallkolloidbildner kommen Verbindungen der Metalle Au, Ag, As, Bi, Nb, Cu, Fe, Pd, Pt, Sb und Sn infrage. Bei den Glaskeramiken handelt es sich insbesondere um Lithium-Aluminosilikat-Glaskeramiken oder Magnesium-Aluminosilikat-Glaskeramiken, die hohe Mengen an Aluminiumoxid von mindestens 18,0 Gew.-% sowie signifikante Mengen an gesundheitsschädlichem Antimon- und Arsenoxid enthalten.

Die WO 03/050053 und die WO 03/050051 beschreiben antimikrobiell wirkende Glaskeramik-Pulvers, die im Bereich der Zahnpflege zum Beispiel als Bestandteil von Mundwasser, Zahnpasta oder Zahnseide eingesetzt werden können. Zur Verstärkung der antimikrobiellen Eigenschaften können antimikrobiell wirkende Ionen wie zum Beispiel Ag, Au, I, Ce, Cu, Zn und Sn vorhanden sein. Die Glaskeramiken weisen als Hauptkristallphase Alkali-Erdalkali-Silikate und/oder Erdalkalisilikate, insbesondere NaCa-Silikate und Ca-Silikate, auf.

Die WO 2005/058768 offenbart Körper aus Lithium-Aluminosilikat-Glaskeramiken, die sich insbesondere zur Herstellung von Kochfeldern eignen. Die Körper weisen eine Oberflächenschicht mit höherem Gehalt an kristallisationsfördernden chemischen Elementen aus der Gruppe von Zn, Cu, Zr, La, Nb, Y, Ti, Ge, V und Sn auf. Als Hauptkristallphase enthalten die Glaskeramiken eine Hochquarz-Mischkristallphase.

Die EP 1 688 397 beschreibt Lithiumsilikat-Glaskeramiken, die geringe Mengen an Zinkoxid sowie hohe Mengen von 2,0 bis 5,0 Gew.-% Keimbildungsmittel enthalten. Das Keimbildungsmittel zur Bildung von Lithiummetasilikat ist insbesondere ausgewählt aus P₂O₅ und Verbindungen der Elemente Pt, Ag, Cu und W und es ist bevorzugt P₂O₅. Demgemäß wird auch bei allen konkret angegebenen Glaskeramiken als Keimbildungsmittel P₂O₅ eingesetzt, was neben Lithiumsilikat ebenfalls zur Bildung von Lithiumphosphat als Kristallphase führt. Durch Lithiumphosphat-Kristalle können allerdings die mechanischen und/oder optischen Eigenschaften von Lithiumsilikat-Glaskeramiken beeinträchtigt werden.

Die WO 2013/053866 beschreibt Lithiumsilikat-Glaskeramiken mit Gehalt an tetravalenten Metalloxiden, wie Zinnoxid. Als Keimbildner zur Bildung von Lithiumsilikat werden Metalle und insbesondere Ag, Au, Pt und Pd und besonders bevorzugt P₂O₅ verwendet. Beim Einsatz von P₂O₅ als Keimbildner kommt es jedoch zur Bildung von unerwünschtem Lithiumphosphat als Kristallphase. Weiter enthalten die Glaskeramiken nur sehr geringe Mengen an den einwertigem Metalloxiden K₂O und Na₂O und sind bevorzugt im Wesentlichen frei von diesen Metalloxiden.

Die EP 3 696 149 A1 beschreibt fluoreszierende Glaskeramiken und Gläser, die zur Erzeugung von Fluoreszenz Cer und Zinn und als Keimbildner P₂O₅ enthalten. Dabei dient das Zinn zur gewünschten Einstellung des Gleichgewichts zwischen Ce³⁺- und Ce⁴⁺-Ionen, wodurch die gewünschte Fluoreszenz und die gewünschte Einfärbung der Glaskeramik erzielt werden. Durch die Verwendung von P₂O₅ als Keimbildner können wiederum unerwünschte Phosphat-Kristallphasen in den Glaskeramiken vorhanden sein.

Insgesamt verfügen die bekannten Glaskeramiken nicht über die für ein dentales Restaurationsmaterial wünschenswerten Eigenschaften oder sie enthalten hohe Mengen an P₂O₅, was zur Bildung von unerwünschten Kristallphasen, wie Phosphatphasen oder Cristobalit, führen kann, welche ihrerseits insbesondere die für ein Restaurationsmaterial gewünschten mechanischen und/oder optischen Eigenschaften beeinträchtigen können.

Der Erfindung liegt daher die Aufgabe zugrunde, eine Glaskeramik bereitzustellen, die über eine Kombination von sehr guten mechanischen und optischen Eigenschaften verfügt. Die Glaskeramik soll weiter in einfacher Weise zu dentalen Restaurationen verarbeitbar sein und sich damit ausgezeichnet als restauratives Dentalmaterial eignen.

Diese Aufgabe wird durch die Lithiumsilikat-Glaskeramik nach den Ansprüchen 1 bis 12 und 15 gelöst. Gegenstand der Erfindung sind ebenfalls das Ausgangsglas nach den Ansprüchen 13 bis 15, die Verfahren nach den Ansprüchen 16, 17 und 20 sowie die Verwendung nach den Ansprüchen 18 und 19.

Die erfindungsgemäße Lithiumsilikat-Glaskeramik zeichnet sich dadurch aus, dass sie 0,01 bis 4,5, bevorzugt 0,03 bis 3,0, besonders bevorzugt 0,1 bis 2,0 und ganz besonders bevorzugt 0,2 bis 1,5 Gew.-% Zinn, berechnet als SnO₂, enthält.

Die erfindungsgemäße Glaskeramik zeigt überraschenderweise eine vorteilhafte Kombination von für ein restauratives Dentalmaterial wünschenswerten mechanischen und optischen Eigenschaften. Die Glaskeramik hat eine hohe Festigkeit und Bruchzähigkeit und ihr kann in einfacher Weise durch insbesondere maschinelle Bearbeitung die Form einer Dentalrestauration gegeben werden.

Dabei ist es überraschend, dass für die Erzielung dieser Eigenschaften der Einsatz von P₂O₅ als üblichem Keimbildner für Lithiumsilikat-Glaskeramiken nicht erforderlich ist. Es wird angenommen, dass in der erfindungsgemäßen Glaskeramik das vorhandene Zinn als Keimbildner dient. Es ist besonders überraschend, dass auch bereits geringe Mengen an Zinn wirksam sind.

Auch kann die erfindungsgemäße Glaskeramik sehr hohe Gehalte an Lithiumsilikat-Kristallphasen von z.B. mehr als 65 Gew.-% aufweisen, und es wird wiederum angenommen, dass hierfür im Wesentlichen das vorhandene Zinn als Keimbildner verantwortlich ist. Derart hohe Gehalte an Lithiumsilikat-Kristallphasen sind bei der Verwendung von P₂O₅ als Keimbildner regelmäßig nicht erzeugbar.

Die erfindungsgemäße Glaskeramik weist zudem vorzugsweise nur geringe Mengen an weiteren Kristallphasen, z.B. Lithiumphosphat oder Cristobalit, auf. Die Bildung großer Mengen an solchen weiteren Kristallphasen tritt bei der bisher üblichen Verwendung von großen Mengen an P₂O₅ als Keimbildner häufig auf, und diese weiteren Kristallphasen können einen negativen Effekt auf die mechanischen und/oder optischen Eigenschaften von Lithiumsilikat-Glaskeramiken haben. Auch wird Lithium durch die Bildung von Lithiumphosphat-Kristallen verbraucht und steht somit nicht mehr für die Bildung von Lithiumsilikat zur Verfügung. Gerade das Lithiumsilikat spielt eine wesentliche Rolle insbesondere für die ausgezeichneten mechanischen Eigenschaften von Lithiumsilikat-Glaskeramiken. Demnach ist die erfindungsgemäße Glaskeramik auch in dieser Hinsicht von Vorteil.

Die erfindungsgemäße Glaskeramik enthält insbesondere 65,0 bis 89,0, bevorzugt 68,0 bis 83,0, besonders bevorzugt 75,0 bis 81,0 und ganz besonders bevorzugt 77,0 bis 80,0 Gew.-% SiO₂.

Es ist weiter bevorzugt, dass die erfindungsgemäße Glaskeramik 10,0 bis 21,0, bevorzugt 11,0 bis 20,0, besonders bevorzugt 13,0 bis 19,0 und ganz besonders bevorzugt 14,0 bis 18,0 Gew.-% Li₂O enthält. Es wird angenommen, dass Li₂O auch die Viskosität der Glasmatrix erniedrigt und damit die Kristallisation der gewünschten Kristallphasen fördert.

Auch ist es bevorzugt, dass die Glaskeramik 0 bis 7,0 und bevorzugt 1,0 bis 6,0 Gew.-% Oxid einwertiger Elemente Me^{I}₂O ausgewählt aus der Gruppe von K₂O, Na₂O, Rb₂O, Cs₂O und Mischungen davon enthält.

Besonders bevorzugt enthält die Glaskeramik mindestens eines und insbesondere alle der folgenden Oxide einwertiger Elemente Me^{I}₂O in den angegebenen Mengen:

| Komponente | Gew.-% |
|---|---|
| K₂O | 0 bis 6,0 |
| Na₂O | 0 bis 6,0 |
| Rb₂O | 0 bis 5,0 |
| Cs₂O | 0 bis 4,0 |

In einer besonders bevorzugten Ausführungsform enthält die erfindungsgemäße Glaskeramik 1,0 bis 5,0, bevorzugt 1,2 bis 4,5, besonders bevorzugt 1,5 bis 4,0 und ganz besonders bevorzugt 1,5 bis 2,5 Gew.-% K₂O.

Des Weiteren ist es bevorzugt, dass die Glaskeramik 0 bis 15,0, bevorzugt 0 bis 10,0 und besonders bevorzugt 0 bis 8,0 Gew.-% Oxid zweiwertiger Elemente Me^{II}O ausgewählt aus der Gruppe von CaO, MgO, SrO, ZnO und Mischungen davon enthält.

In einer weiteren bevorzugten Ausführungsform enthält die Glaskeramik weniger als 2,0 Gew.-% an BaO. Die Glaskeramik ist insbesondere im Wesentlichen frei von BaO.

Bevorzugt enthält die Glaskeramik mindestens eines und insbesondere alle der folgenden Oxide zweiwertiger Elemente Me^{II}O in den angegebenen Mengen:

| Komponente | Gew.-% |
|---|---|
| CaO | 0 bis 10,0, insbesondere 0 bis 8,0 |
| MgO | 0 bis 8,0, insbesondere 0 bis 6,0 |
| SrO | 0 bis 15,0, insbesondere 0 bis 12,0 |
| ZnO | 0 bis 12,0, insbesondere 0 bis 10,0 |

Es ist weiter eine Glaskeramik bevorzugt, die 0 bis 12,0, bevorzugt 0,1 bis 10,0 und besonders bevorzugt 1,0 bis 8,0 Gew.-% Oxid dreiwertiger Elemente Me^{III}₂O₃ ausgewählt aus der Gruppe von Al₂O₃, B₂O₃, Y₂O₃, La₂O₃ und Mischungen davon enthält.

Besonders bevorzugt enthält die Glaskeramik mindestens eines und insbesondere alle der folgenden Oxide dreiwertiger Elemente Me^{III}₂O₃ in den angegebenen Mengen:

| Komponente | Gew.-% |
|---|---|
| Al₂O₃ | 0 bis 6,0 |
| B₂O₃ | 0 bis 5,0 |
| Y₂O₃ | 0 bis 8,5 |
| La₂O₃ | 0 bis 11,5 |
| Ga₂O₃ | 0 bis 5,0 |
| In₂O₃ | 0 bis 5,0 |

In einer besonders bevorzugten Ausführungsform enthält die Glaskeramik 0,1 bis 6,0, bevorzugt 1,0 bis 5,0, besonders bevorzugt 1,5 bis 4,0 und ganz besonders bevorzugt 1,5 bis 3,0 Gew.-% Al₂O₃.

Ferner ist eine Glaskeramik bevorzugt, die 0 bis 9,0 und besonders bevorzugt 0 bis 7,0 Gew.-% Oxid vierwertiger Elemente Me^{IV}O₂ ausgewählt aus der Gruppe von ZrO₂, TiO₂, GeO₂ und Mischungen davon enthält.

Besonders bevorzugt enthält die Glaskeramik mindestens eines und insbesondere alle der folgenden Oxide vierwertiger Elemente Me^{IV}O₂ in den angegebenen Mengen:

| Komponente | Gew.-% |
|---|---|
| ZrO₂ | 0 bis 9,0 |
| TiO₂ | 0 bis 6,0 |
| GeO₂ | 0 bis 4,0 |

In einer weiteren bevorzugten Ausführungsform enthält die Glaskeramik 0 bis 10,0 und bevorzugt 0 bis 8,0 Gew.-% Oxid fünfwertiger Elemente Me^{V}₂O₅ ausgewählt aus der Gruppe von Ta₂O₅ und Nb₂O₅ und Mischungen davon.

Besonders bevorzugt enthält die Glaskeramik mindestens eines und insbesondere alle der folgenden Oxide fünfwertiger Elemente Me^{V}₂O₅ in den angegebenen Mengen:

| Komponente | Gew.-% |
|---|---|
| Ta₂O₅ | 0 bis 8,0 |
| Nb₂O₅ | 0 bis 10,0 |

Es ist auch bevorzugt, dass die erfindungsgemäße Glaskeramik weniger als 3,0, bevorzugt weniger als 2,0, besonders bevorzugt weniger als 1,0 und ganz besonders bevorzugt weniger als 0,1 Gew.-% P₂O₅ enthält. In einer weiter bevorzugten Ausführungsform ist die Glaskeramik im Wesentlichen frei von P₂O₅.

In einer weiteren Ausführungsform enthält die Glaskeramik 0 bis 7,0 und bevorzugt 0 bis 6,0 Gew.-% Oxid sechswertiger Element Me^{VI}O₃ ausgewählt aus der Gruppe von WO₃, MoO₃ und Mischungen davon.

Besonders bevorzugt enthält die Glaskeramik mindestens eines und insbesondere alle der folgenden Oxide Me^{VI}O₃ in den angegebenen Mengen:

| Komponente | Gew.-% |
|---|---|
| WO₃ | 0 bis 4,5 |
| MoO₃ | 0 bis 5,5 |

In einer weiteren Ausführungsform enthält die erfindungsgemäße Glaskeramik 0 bis 1,0 und insbesondere 0 bis 0,5 Gew.-% Fluor.

Besonders bevorzugt ist eine Glaskeramik, die mindestens eine und bevorzugt alle der folgenden Komponenten in den angegebenen Mengen enthält:

| Komponente | Gew.-% |
|---|---|
| SiO₂ | 65,0 bis 89,0 |
| Li₂O | 10,0 bis 21,0 |
| Zinn, berechnet als SnO₂, | 0,01 bis 4,5 |
| P₂O₅ | weniger als 3,0 |
| Me^{I}₂O | 0 bis 7,0 |
| Me^{II}O | 0 bis 15,0 |
| Me^{III}₂O₃ | 0 bis 12,0 |
| Me^{IV}O₂ | 0 bis 9,0 |
| Me^{V}₂O₅ | 0 bis 10,0 |
| Me^{VI}O₃ | 0 bis 7,0 |
| Fluor | 0 bis 1,0, |

wobei Me^{I}₂O, Me^{II}O, Me^{III}₂O₃, Me^{IV}O₂, Me^{V}₂O₅ und Me^{VI}O₃ die oben angegebene Bedeutung haben.

In einer weiteren besonders bevorzugten Ausführungsform enthält die Glaskeramik mindestens eine und bevorzugt alle der folgenden Komponenten in den angegebenen Mengen:

| Komponente | Gew.-% |
|---|---|
| SiO₂ | 68,0 bis 83,0 |
| Li₂O | 11,0 bis 20,0 |
| Zinn, berechnet als SnO₂, | 0,03 bis 3,0 |
| P₂O₅ | weniger als 2,0 |
| K₂O | 0 bis 6, 0 |
| Na₂O | 0 bis 6,0 |
| Rb₂O | 0 bis 5,0 |
| Cs₂O | 0 bis 4,0 |
| CaO | 0 bis 10,0 |
| MgO | 0 bis 8,0 |
| SrO | 0 bis 15,0 |
| ZnO | 0 bis 12.0 |
| Al₂O₃ | 0 bis 6,0 |
| B₂O₃ | 0 bis 5,0 |
| Y₂O₃ | 0 bis 8,5 |
| La₂O₃ | 0 bis 11,5 |
| Ga₂O₃ | 0 bis 5,0 |
| In₂O₃ | 0 bis 5,0 |
| ZrO₂ | 0 bis 9,0 |
| TiO₂ | 0 bis 6,0 |
| GeO₂ | 0 bis 4,0 |
| Ta₂O₅ | 0 bis 8,0 |
| Nb₂O₅ | 0 bis 10,0 |
| WO₃ | 0 bis 4,5 |
| MoO₃ | 0 bis 5,5 |
| Fluor | 0 bis 0,5. |

Manche der vorstehend genannten Komponenten können als Färbemittel und/oder Fluoreszenzmittel dienen. Die erfindungsgemäße Glaskeramik kann darüber hinaus noch weitere Färbemittel und/oder Fluoreszenzmittel enthalten. Diese können insbesondere aus weiteren anorganischen Pigmenten und/oder Oxiden von d- und f-Elementen, wie z.B. den Oxiden von Mn, Fe, Co, Pr, Nd, Tb, Er, Dy, Eu und Yb, oder Metallen, bevorzugt Ag, Cu und Au, ausgewählt sein.

In einer bevorzugten Ausführungsform der Glaskeramik liegt das Molverhältnis von SiO₂ zu Li₂O im Bereich von 1,5 bis 4,0, bevorzugt 1,7 bis 3,5 und besonders bevorzugt 2,0 bis 3,0.

Es ist weiter bevorzugt, dass die erfindungsgemäße Glaskeramik Lithiumdisilikat oder Lithiummetasilikat als Hauptkristallphase und insbesondere Lithiumdisilikat als Hauptkristallphase enthält.

Mit dem Begriff "Hauptkristallphase" wird die Kristallphase bezeichnet, die von allen in der Glaskeramik vorhandenen Kristallphasen den höchsten Gewichtsanteil hat. Die Bestimmung der Mengen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Es ist bevorzugt, dass die erfindungsgemäße Glaskeramik mindestens 1,0 Gew.-%, bevorzugt mindestens 1,5 Gew.-% und besonders bevorzugt mindestens 2,0 Gew.-% Lithiummetasilikat-Kristalle aufweist. Besonders bevorzugt enthält die erfindungsgemäße Glaskeramik 1,0 bis 50,0 Gew.-%, bevorzugt 1,5 bis 45,0 Gew.-% und besonders bevorzugt 2,0 bis 40,0 Gew.-% Lithiummetasilikat-Kristalle.

In einer anderen Ausführungsform ist es bevorzugt, dass die erfindungsgemäße Glaskeramik mindestens 50,0 Gew.-%, bevorzugt mindestens 55,0 Gew.-% und besonders bevorzugt mindestens 60,0 Gew.-% Lithiumdisilikat-Kristalle aufweist. Besonders bevorzugt enthält die erfindungsgemäße Glaskeramik 50,0 bis 90,0 Gew.-%, bevorzugt 55,0 bis 85,0 Gew.-% und besonders bevorzugt 60,0 bis 80,0 Gew.-% Lithiumdisilikat-Kristalle.

Die erfindungsgemäße Glaskeramik zeichnet sich durch besonders gute mechanische und optische Eigenschaften aus und sie kann durch Wärmebehandlung eines entsprechenden Ausgangsglases oder eines entsprechenden Ausgangsglases mit Keimen gebildet werden. Diese Materialien können daher als Vorstufen für die erfindungsgemäße Glaskeramik dienen.

Die Art und insbesondere die Menge der gebildeten Kristallphasen können durch die Zusammensetzung des Ausgangsglases sowie die Wärmebehandlung gesteuert werden, die zur Herstellung der Glaskeramik aus dem Ausgangsglas angewendet wird. Die Beispiele veranschaulichen dies anhand der Variation der Zusammensetzung des Ausgangsglases und der angewendeten Wärmebehandlung.

Die Glaskeramik weist eine hohe biaxiale Bruchfestigkeit von vorzugsweise mindestens 150 MPa und besonders bevorzugt mindestens 250 MPa auf. Die biaxiale Bruchfestigkeit wurde gemäß ISO 6872 (2008) (Kolben-auf-drei-Kugeln-Prüfung) bestimmt.

Die Glaskeramik weist zudem eine hohe Bruchzähigkeit von vorzugsweise mindestens 1,5 MPa ·m^{0,5}, besonders bevorzugt mindestens 2,0 MPa·m^{0,5} und ganz besonders bevorzugt mindestens 2,5 MPa·m^{0,5} auf. Die Bruchzähigkeit wurde gemäß ISO 6872 (2015) (SEVNB-Methode) bestimmt.

Weiter hat die Glaskeramik eine hohe chemische Stabilität gemessen als Säurelöslichkeit nach ISO 6872 (2015) von vorzugsweise weniger als 100 g/cm².

Die bei der erfindungsgemäßen Glaskeramik vorliegende besondere Kombination an Eigenschaften erlaubt es sogar, sie als Dentalmaterial und insbesondere als Material zur Herstellung von Dentalrestaurationen einzusetzen.

Die Erfindung betrifft ebenfalls Vorstufen mit entsprechender Zusammensetzung, aus denen die erfindungsgemäße Glaskeramik durch Wärmebehandlung hergestellt werden können. Diese Vorstufen sind ein entsprechend zusammengesetztes Ausgangsglas und ein entsprechend zusammengesetztes Ausgangsglas mit Keimen. Die Bezeichnung "entsprechender Zusammensetzung" bedeutet, dass diese Vorstufen die gleichen Komponenten in den gleichen Mengen wie die Glaskeramik enthalten, wobei die Komponenten wie bei Gläsern und Glaskeramiken üblich mit Ausnahme von Fluor als Oxide berechnet werden.

Die Erfindung betrifft daher ebenfalls ein Ausgangsglas, das die Komponenten der erfindungsgemäßen Glaskeramik enthält.

Das erfindungsgemäße Ausgangsglas enthält daher insbesondere geeignete Mengen an SiO₂, Li₂O und Zinn, die zur Ausbildung der erfindungsgemäßen Glaskeramik erforderlich sind. Weiter kann das Ausgangsglas auch noch andere Komponenten enthalten, wie sie oben für die erfindungsgemäße Glaskeramik angegeben sind. Es sind alle solche Ausführungsformen für die Komponenten des Ausgangsglases bevorzugt, die auch für die Komponenten der erfindungsgemäßen Glaskeramik als bevorzugt angegeben sind.

Besonders bevorzugt liegt das Ausgangsglas in Form eines monolithischen Rohlings vor, der durch Gießen einer Schmelze des Ausgangsglases in eine Form erhalten wird.

Die Erfindung betrifft ebenfalls ein solches Ausgangsglas, das Keime für die Kristallisation von Lithiumsilikat, insbesondere Lithiummetasilikat und/oder Lithiumdisilikat, enthält.

Die Herstellung des Ausgangsglases erfolgt insbesondere in der Weise, dass eine Mischung von geeigneten Ausgangsmaterialien, wie z.B. Carbonaten, Oxiden und Halogeniden, bei Temperaturen von insbesondere etwa 1500 bis 1800°C für 0,5 bis 4 h geschmolzen wird. Als Ausgangsmaterial für Zinn können insbesondere SnO oder SnO₂ verwendet werden. Die Schmelze kann dann in Wasser gegossen werden, um eine Fritte herzustellen. Zur Erzielung einer besonders hohen Homogenität wird die erhaltene Glasfritte erneut aufgeschmolzen.

Die Schmelze kann dann in Formen gegossen werden, um Rohlinge des Ausgangsglases, sogenannte Massivglasrohlinge oder monolithische Rohlinge, zu erzeugen.

Durch Wärmebehandlung des Ausgangsglases kann zunächst die weitere Vorstufe Ausgangsglas mit Keimen erzeugt werden. Durch Wärmebehandlung dieser weiteren Vorstufe kann dann die erfindungsgemäße Lithiumsilikat-Glaskeramik erzeugt werden. Alternativ kann die erfindungsgemäße Glaskeramik durch Wärmebehandlung des Ausgangsglases gebildet werden.

Es ist bevorzugt, das Ausgangsglas einer Wärmebehandlung bei einer Temperatur von 400 bis 600°C, insbesondere 430 bis 550°C und besonders bevorzugt 440 bis 520°C für eine Dauer von bevorzugt 5 bis 120 min, insbesondere 10 bis 60 min, zu unterwerfen, um das Ausgangsglas mit Keimen für die Kristallisation von Lithiumsilikat zu erzeugen.

Es ist weiter bevorzugt, das Ausgangsglas oder das Ausgangsglas mit Keimen einer Wärmebehandlung bei einer Temperatur von 800 bis 1050°C, vorzugsweise 850 bis 1020°C, für eine Dauer von insbesondere 5 Sekunden bis 120 min, bevorzugt 1 min bis 100 min, besonders bevorzugt 5 min bis 60 min und weiter bevorzugt 10 min bis 30 min, zu unterwerfen, um die erfindungsgemäße Glaskeramik herzustellen.

Die Erfindung betrifft daher ebenfalls ein Verfahren zur Herstellung der erfindungsgemäßen Glaskeramik, bei dem das Ausgangsglas oder das Ausgangsglas mit Keimen mindestens einer Wärmebehandlung im Bereich von 800 bis 1050°C, vorzugsweise 850 bis 1020°C, für eine Dauer von insbesondere 5 Sekunden bis 120 min, bevorzugt 1 min bis 100 min, besonders bevorzugt 5 min bis 60 min und weiter bevorzugt 10 min bis 30 min, unterzogen wird.

Die im erfindungsgemäßen Verfahren durchgeführte mindestens eine Wärmebehandlung kann auch im Rahmen eines Heißpressens, insbesondere eines Massivglasrohlings, oder Sinterns, insbesondere eines Pulvers, des erfindungsgemäßen Ausgangsglases oder des erfindungsgemäßen Ausgangsglases mit Keimen erfolgen.

In einer weiteren bevorzugten Ausführungsform kann das Ausgangsglas oder das Ausgangsglas mit Keimen zunächst einer Wärmebehandlung bei einer Temperatur von 550 bis 800°C, vorzugsweise 600 bis 800°C, für eine Dauer von insbesondere 5 Sekunden bis 120 min, bevorzugt 1 min bis 100 min, besonders bevorzugt 5 min bis 60 min und weiter bevorzugt 10 min bis 30 min, unterworfen werden, um die erfindungsgemäße Glaskeramik mit Lithiummetasilikat als Hauptkristallphase zu erzeugen.

Die erfindungsgemäße Glaskeramik mit Lithiummetasilikat als Hauptkristallphase kann dann einer weiteren Wärmebehandlung unterworfen werden, um Lithiummetasilikatkristalle in Lithiumdisilikatkristalle umzuwandeln und insbesondere die erfindungsgemäße Glaskeramik mit Lithiumdisilikat als Hauptkristallphase zu bilden. Vorzugsweise wird dabei die Glaskeramik einer weiteren Wärmebehandlung bei einer Temperatur von 800 bis 1050°C, bevorzugt 850 bis 1020°C und besonders bevorzugt 900 bis 1020°C, insbesondere für eine Dauer von 5 Sekunden bis 120 min, bevorzugt 1 min bis 100 min, besonders bevorzugt 1 min bis 60 min, weiter bevorzugt 5 bis 30 min und ganz besonders bevorzugt 5 bis 20 min, unterworfen.

Die geeigneten Bedingungen für eine gegebene Glaskeramik können beispielsweise bestimmt werden, indem Röntgenbeugungsanalysen bei unterschiedlichen Temperaturen durchgeführt werden.

Die erfindungsgemäßen Glaskeramiken und die erfindungsgemäßen Gläser liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden, z.B. zu dentalen Restaurationen. Sie können aber auch in Form von dentalen Restaurationen, wie Inlays, Onlays, Kronen, Veneers, Schalen oder Abutments, vorliegen.

Aus den erfindungsgemäßen Glaskeramiken und den erfindungsgemäßen Gläsern können dentale Restaurationen, wie Brücken, Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass der Glaskeramik oder dem Glas durch Verpressen und insbesondere durch maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das erfindungsgemäße Ausgangsglas, das erfindungsgemäße Ausgangsglas mit Keimen und die erfindungsgemäße Glaskeramik verwendet werden. Dabei können die erfindungsgemäßen Gläser und Glaskeramiken insbesondere in Form von Rohlingen in beliebiger Form und Größe eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Ausgangsglas, das erfindungsgemäße Ausgangsglas mit Keimen und die erfindungsgemäße Glaskeramik verwendet werden. Bevorzugt werden das Ausgangsglas mit Keimen oder die erfindungsgemäße Glaskeramik mit Lithiummetasilikat als Hauptkristallphase verwendet. Dabei können die erfindungsgemäßen Gläser und Glaskeramiken insbesondere in Form von Rohlingen eingesetzt werden.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser als Dentalmaterial und bevorzugt zur Herstellung dentaler Restaurationen, wie Brücken, Inlays, Onlays, Veneers, Abutments, Teilkronen, Kronen oder Schalen.

Die Erfindung betrifft mithin auch ein Verfahren zur Herstellung einer dentalen Restauration, insbesondere Brücke, Inlay, Onlay, Veneer, Abutment, Teilkrone, Krone oder Schale, bei dem der erfindungsgemäßen Glaskeramik oder dem erfindungsgemäßen Glas durch Verpressen oder durch maschinelle Bearbeitung, insbesondere im Rahmen eines CAD/CAM-Verfahrens, die Form der gewünschten dentalen Restauration gegeben wird.

Die Erfindung wird im Folgenden anhand von sie nicht-beschränkenden Beispielen näher erläutert.

### Beispiele

### Beispiele 1 bis 49 - Zusammensetzung und Kristallphasen

Es wurden insgesamt 49 erfindungsgemäße Gläser und Glaskeramiken mit der aus Tabelle I angegebenen Zusammensetzung über Erschmelzung entsprechender Ausgangsmaterialien zur Erzeugung von Ausgangsgläsern und deren anschließende Wärmebehandlung zur gesteuerten Kristallisation hergestellt.

Die angewendeten Wärmebehandlungen sowie Eigenschaften der erhaltenen Glaskeramiken sind ebenfalls in Tabelle I angegeben. Dabei bedeuten
- T_{g}: Glasübergangstemperatur, bestimmt mittels DSC
- T_{S} und t_{S}: Angewendete Temperatur und Zeit für Erschmelzung des Ausgangsglases
- T_{Kb} und t_{Kb}: Angewendete Temperatur und Zeit für Keimbildung des Ausgangsglases
- T_{C1} und t_{C1}: Angewendete Temperatur und Zeit für die erste Kristallisation
- T_{C2} und t_{C2}: Angewendete Temperatur und Zeit für die zweite Kristallisation
- K_{IC}: Bruchzähigkeit, gemessen gemäß ISO 6872 (2015) (SEVNB-Methode)
- Chem. Stabilität: Gemessen als Masseverlust gemäß ISO 6872 (2015)
- σ_{Biax}: Biaxiale Bruchfestigkeit, gemessen gemäß ISO 6872 (2015) (Kolben-auf-drei-Kugeln-Test)

In den Beispielen wurden zunächst Ausgangsgläser mit den in der Tabelle I angegebenen Zusammensetzungen im 100 bis 200 g Maßstab aus üblichen Rohstoffen bei der Temperatur Tₛ für die Dauer tₛ erschmolzen, wobei das Erschmelzen sehr gut ohne Bildung von Blasen oder Schlieren möglich war. Durch Eingießen der Ausgangsgläser in Wasser wurden Glasfritten hergestellt, die gegebenenfalls zur Homogenisierung anschließend ein zweites Mal bei der Temperatur Tₛ für die Dauer tₛ erschmolzen wurden. Die erhaltenen Schmelzen des Ausgangsglases wurden anschließend in eine Graphitform gegossen, um monolithische Glasblöcke zu erzeugen.

Eine erste Wärmebehandlung der so erhaltenen Glasblöcke bei der Temperatur T_{Kb} für die Dauer t_{Kb} führte zur Entspannung der Gläser und Bildung von Gläsern mit Keimen. Diese keimhaltigen Gläser kristallisierten durch eine weitere Wärmebehandlung bei der Temperatur T_{C1} für die Dauer t_{C1} zu Glaskeramiken mit Lithiummetasilikat oder Lithiumdisilikat als Hauptkristallphase, wie durch Röntgenbeugungsuntersuchungen bei Raumtemperatur festgestellt wurde. In einigen Fällen wurde anschließend noch eine weitere Wärmebehandlung bei der Temperatur T_{C2} für die Dauer t_{C2} durchgeführt, die zu Glaskeramiken mit Lithiumdisilikat als Hauptkristallphase führte.

Bei den Beispielen 9 und 38 wurden durch Eingießen der Ausgangsgläser in Wasser Glasfritten hergestellt. Diese Fritten wurde zerkleinert, gesiebt und anschliessend bei der in der Tabelle I angegebenen Temperatur und Zeit gesintert.

Die Mengen der Kristallphasen wurden mittels Röntgenbeugung bestimmt. Dazu wurden durch Mahlen und Sieben (< 45 µm) Pulver der jeweiligen Glaskeramik hergestellt und mit Al₂O₃ (Alfa Aesar, Produkt-Nr. 42571) als internem Standard im Verhältnis von 80 Gew.-% Glaskeramik zu 20 Gew.-% Al₂O₃ gemischt. Diese Mischung wurde mit Aceton aufgeschlämmt, um eine möglichst gute Durchmischung zu erreichen. Anschließend wurde die Mischung bei etwa 80°C getrocknet. Danach wurde mittels eines D8 Advance Diffraktometers der Firma Bruker ein Diffraktogramm im Bereich 10 bis 100° 2θ mittels CuKα-Strahlung und einer Schrittweite von 0,014° 2θ aufgenommen. Dieses Diffraktogramm wurde dann mit der Software TO-PAS 5.0 der Firma Bruker nach der Rietveld-Methode ausgewertet. Durch Vergleich der Intensitäten der Peaks mit denen von Al₂O₃ wurden die Phasenanteile bestimmt.

Zur Bestimmung der biaxialen Bruchfestigkeiten gemäß ISO 6872 (2015) (Kolben-auf-drei-Kugeln-Prüfung) wurden Halter auf Blöcke der entspannten und keimgebildeten Gläser geklebt und diese Blöcke wurden anschließend mittels einer CAD/CAM-Schleifeinheit (Sirona InLab) bearbeitet. Die schleifende Bearbeitung erfolgte mittels diamantbeschichteter Schleifwerkzeuge. Die so erhaltenen Plättchen wurden der in der Tabelle angegebenen Wärmebehandlung, d.h. der ersten Kristallisation und ggf. auch der zweiten Kristallisation, unterzogen und anschließend wurden die kristallisierten Plättchen mittels Diamantscheiben auf eine Dicke von 1,2 ± 0,2 mm poliert. An den so hergestellten Proben wurde die biaxiale Bruchfestigkeit bestimmt.

Es wurden für die erzeugten Glaskeramiken hohe biaxiale Bruchfestigkeiten im Bereich von mehr als 179 bis 524 MPa bestimmt.

Die Bestimmung der Bruchzähigkeiten erfolgte gemäß ISO 6872 (2015) (SEVNB-Methode), und es wurden für die erzeugten Glaskeramiken hohe Bruchzähigkeiten im Bereich von 2,6 bis 3,1 MPa·m^{0,5} bestimmt.

Die Untersuchung der chemischen Beständigkeit erfolgte gemäß ISO 6872 (2015), und die erzeugten Glaskeramiken zeigten eine Säurelöslichkeit von weniger als 100 g/cm².

Aus den erzeugten Gläsern und Glaskeramiken wurden durch CAD/CAM-gestützte maschinelle Bearbeitung dentale Kronen hergestellt, und diese Kronen wurden ggf. noch einer Endkristallisation unter den in der Tabelle I angegeben Bedingungen unterzogen.

### Beispiel 50 - Vergleich

Bei diesem Beispiel wurde durch Erschmelzen der entsprechenden Rohstoffe ein Ausgangsglas ohne Zinn hergestellt und dieses Glas wurde anschließend einer Wärmebehandlung unterzogen, um seine Kristallisation zu bewirken.

Es wurde das gleiche Herstellungsverfahren wie das zur Herstellung der Beispiele 1 bis 49 beschriebene Verfahren angewendet. Die verwendete Zusammensetzung, die durchgeführten Wärmebehandlungen sowie die Eigenschaften der erhaltenen Glaskeramik sind ebenfalls in Tabelle I angegeben.

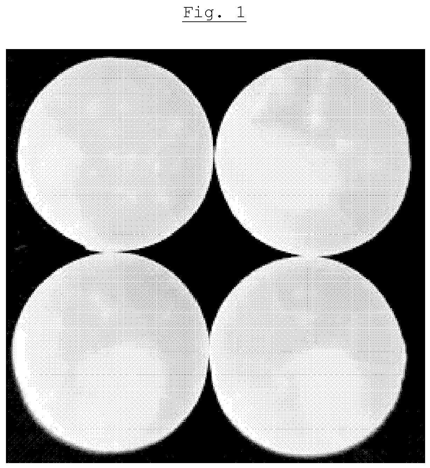

Figur 1 zeigt vier Plättchen der erhaltenen Glaskeramik. Es ist zu erkennen, dass es bei diesen Proben, die kein Zinn enthalten, durch unkontrolliertes Kristallwachstum zur Rissbildung kommt.

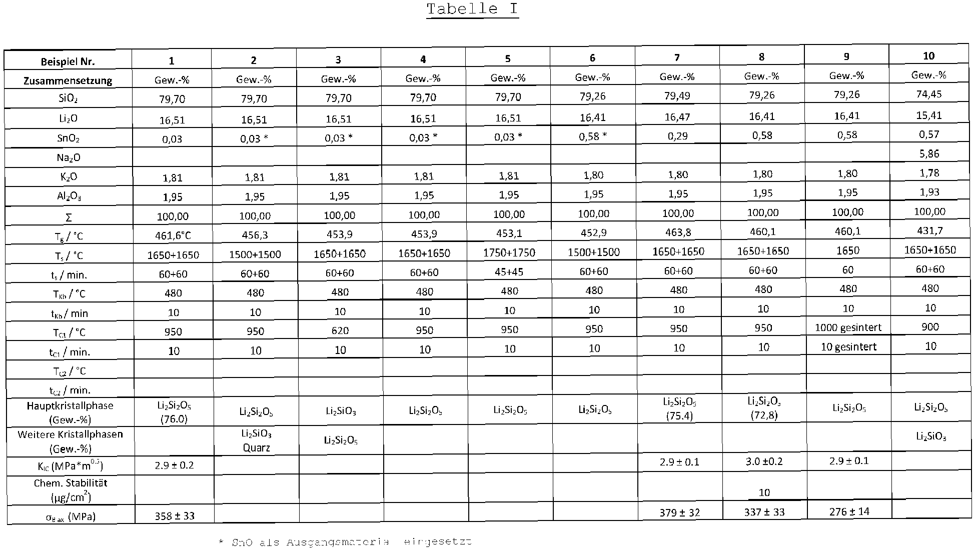

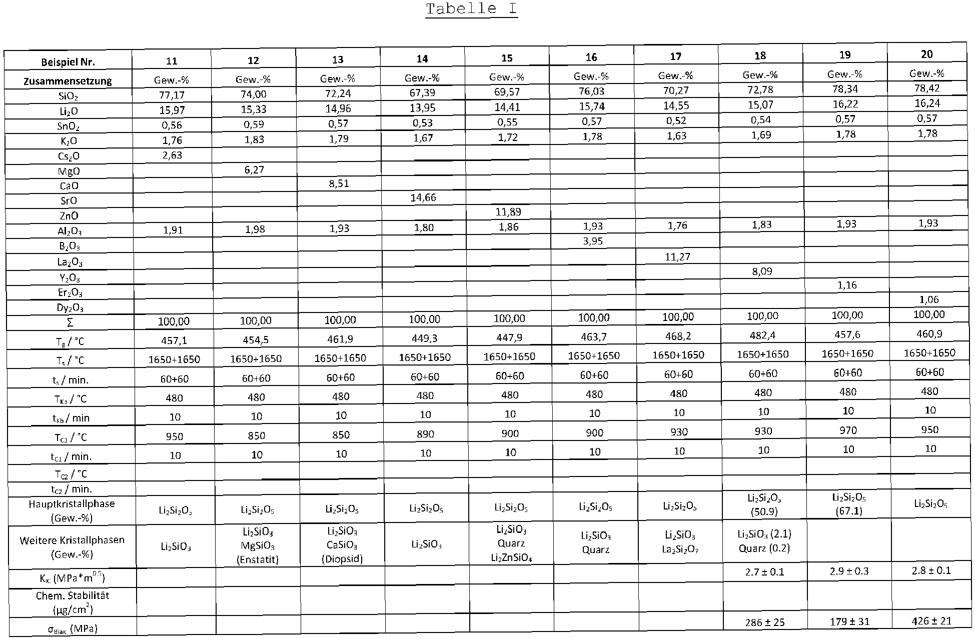

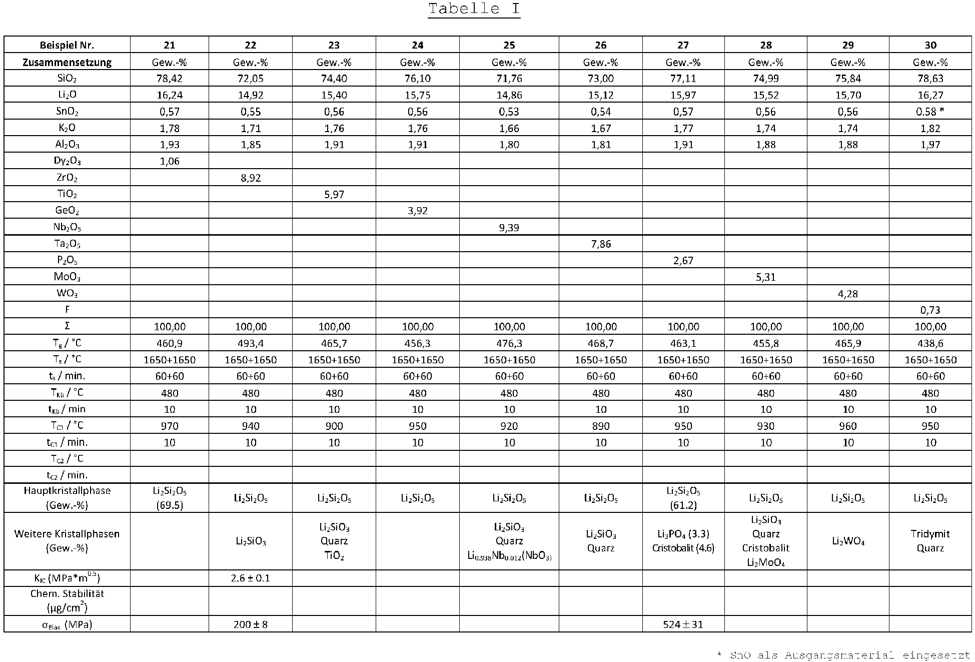

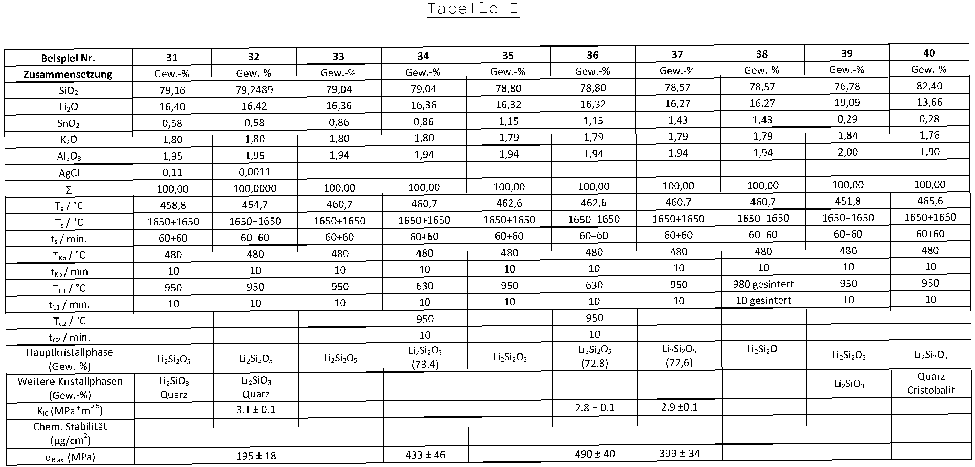

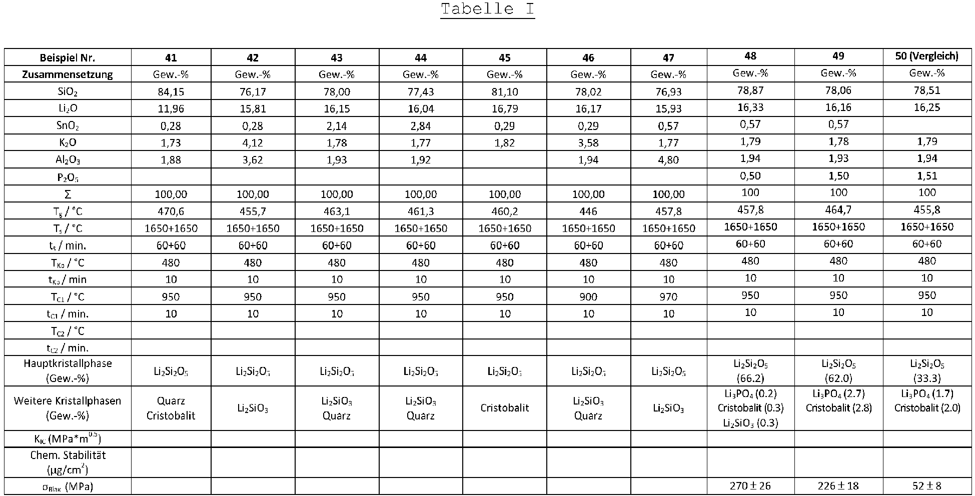

## Patentansprüche

1. Lithiumsilikat-Glaskeramik, die 0,02 bis 4,5, bevorzugt 0,03 bis 3,0, besonders bevorzugt 0,1 bis 2,0 und ganz besonders bevorzugt 0,2 bis 1,5 Gew.-% Zinn, berechnet als SnO₂, enthält.

2. Glaskeramik nach Anspruch 1, die 65,0 bis 89,0, bevorzugt 68,0 bis 83,0, besonders bevorzugt 75,0 bis 81,0 und ganz besonders bevorzugt 77,0 bis 80,0 Gew.-% SiO₂ enthält.

3. Glaskeramik nach Anspruch 1 oder 2, die 10,0 bis 21,0, bevorzugt 11,0 bis 20,0, besonders bevorzugt 13,0 bis 19,0 und ganz besonders bevorzugt 14,0 bis 18,0 Gew.-% Li₂O enthält.

4. Glaskeramik nach einem der Ansprüche 1 bis 3, die weniger als 3,0, bevorzugt weniger als 2,0, besonders bevorzugt weniger als 1,0 und ganz besonders bevorzugt weniger als 0,1 Gew.-% P₂O₅ enthält.

5. Glaskeramik nach einem der Ansprüche 1 bis 4, die 0 bis 7,0 und bevorzugt 1,0 bis 6,0 Gew.-% Oxid einwertiger Elemente Me^{I}₂O ausgewählt aus der Gruppe von K₂O, Na₂O, Rb₂O, Cs₂O und Mischungen davon enthält.

6. Glaskeramik nach einem der Ansprüche 1 bis 5, die 0 bis 6,0, bevorzugt 1,0 bis 5,0, besonders bevorzugt 1,2 bis 4,5, weiter bevorzugt 1,5 bis 4,0 und ganz besonders bevorzugt 1,5 bis 2,5 Gew.-% K₂O enthält

7. Glaskeramik nach einem der Ansprüche 1 bis 6, die 0 bis 15,0, bevorzugt 0 bis 10,0 und besonders bevorzugt 0 bis 8,0 Gew.-% Oxid zweiwertiger Elemente Me^{II}O ausgewählt aus der Gruppe von CaO, MgO, SrO, ZnO und Mischungen davon enthält.

8. Glaskeramik nach einem der Ansprüche 1 bis 7, die 0 bis 12,0, bevorzugt 0,1 bis 10,0 und besonders bevorzugt 1,0 bis 8,0 Gew.-% Oxid dreiwertiger Elemente Me^{III}₂O₃ ausgewählt aus der Gruppe von Al₂O₃, B₂O₃, Y₂O₃, La₂O₃ und Mischungen davon enthält.

9. Glaskeramik nach einem der Ansprüche 1 bis 8, die 0,1 bis 6,0, bevorzugt 1,0 bis 5,0, besonders bevorzugt 1,5 bis 4,0 und ganz besonders bevorzugt 1,5 bis 3,0 Gew.-% Al₂O₃ enthält.

10. Glaskeramik nach einem der Ansprüche 1 bis 9, die Lithiumdisilikat oder Lithiummetasilikat als Hauptkristallphase und bevorzugt Lithiumdisilikat als Hauptkristallphase enthält.

11. Glaskeramik nach einem der Ansprüche 1 bis 10, die 1,0 bis 50,0 Gew.-%, bevorzugt 1,5 bis 45,0 Gew.-% und besonders bevorzugt 2,0 bis 40,0 Gew.-% Lithiummetasilikat-Kristalle aufweist.

12. Glaskeramik nach einem der Ansprüche 1 bis 11, die 50,0 bis 90,0, bevorzugt 55,0 bis 85,0 und besonders bevorzugt 60,0 bis 80,0 Gew.-% Lithiumdisilikat-Kristalle aufweist.

13. Ausgangsglas, das die Komponenten der Glaskeramik nach einem der Ansprüche 1 bis 9 enthält.

14. Ausgangsglas nach Anspruch 13, das Keime für die Kristallisation von Lithiummetasilikat und/oder Lithiumdisilikat enthält.

15. Glaskeramik nach einem der Ansprüche 1 bis 12 oder Ausgangsglas nach Anspruch 13 oder 14, wobei die Glaskeramik und das Ausgangsglas in Form von einem Pulver, einem Granulat, einem Rohling oder einer dentalen Restauration vorliegen.

16. Verfahren zur Herstellung der Glaskeramik gemäß einem der Ansprüche 1 bis 12 oder 15, bei dem das Ausgangsglas gemäß einem der Ansprüche 13 bis 15 mindestens einer Wärmebehandlung, insbesondere im Bereich von 800 bis 1050°C, vorzugsweise 850 bis 1020°C, unterzogen wird.

17. Verfahren nach Anspruch 16, bei dem
(a) das Ausgangsglas einer Wärmebehandlung bei einer Temperatur von 400 bis 600°C, insbesondere 430 bis 550°C und besonders bevorzugt 440 bis 520°C unterworfen wird, um Ausgangsglas mit Keimen zu bilden, und
(b) das Ausgangsglas mit Keimen einer Wärmebehandlung bei einer Temperatur von 800 bis 1050°C, insbesondere 850 bis 1020°C, unterworfen wird, um die Lithiumsilikat-Glaskeramik zu bilden.

18. Verwendung der Glaskeramik gemäß einem der Ansprüche 1 bis 12 oder 15 oder des Ausgangsglases gemäß einem der Ansprüche 13 bis 15 als Dentalmaterial, bevorzugt zur Beschichtung dentaler Restaurationen und besonders bevorzugt zur Herstellung dentaler Restaurationen.

19. Verwendung zur Herstellung dentaler Restaurationen nach Anspruch 18, wobei der Glaskeramik oder dem Ausgangsglas durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

20. Verfahren zur Herstellung einer dentalen Restauration, insbesondere Brücke, Inlay, Onlay, Veneer, Abutment, Teilkrone, Krone oder Schale, bei dem der Glaskeramik gemäß einem der Ansprüche 1 bis 12 oder 15 oder dem Ausgangsglas gemäß einem der Ansprüche 13 bis 15 durch Verpressen oder maschinelle Bearbeitung, insbesondere im Rahmen eines CAD/CAM-Verfahrens, die Form der gewünschten dentalen Restauration gegeben wird.
